# EUROPÄISCHE PATENTSCHRIFT

(11) **EP 3 047 515 B1**
(45) Veröffentlichungstag und Bekanntmachung des Hinweises auf die Patenterteilung: **02.11.2022**
(21) Anmeldenummer: 14772309.2
(22) Anmeldetag: 18.09.2014
(51) Int. Cl.: G01N 33/28, G01N 1/22, G01M 15/10

(54) **MEHRFACH-ÖLEMISSIONSMESSGERÄT FÜR MOTOREN**
MULTIPLE OIL-EMISSION MEASURING DEVICE FOR ENGINES
APPAREIL DE MESURE D'ÉMISSIONS D'HUILE MULTIPLES POUR MOTEURS

(30) Priorität: 20.09.2013 DE 102013218930
(43) Veröffentlichungstag der Anmeldung: 27.07.2016
(73) Patentinhaber: Lubrisense GmbH, 20539 Hamburg (DE)
(72) Erfinder: KRAUSE, Sven, 21227 Bendestorf (DE)
(74) Vertreter: Glawe, Delfs, Moll
(86) Internationale Anmeldenummer: PCT/EP2014/069919
(87) Internationale Veröffentlichungsnummer: WO 2015/040124

(56) Entgegenhaltungen:
- DE-A1-102010 012 606
- ANDREAS BEHN ET AL: "System zur Messung von Ölemissionen im Dieselabgas", MTZ - MOTORTECHNISCHE ZEITSCHRIFT, Bd. 74, Nr. 5, Mai 2013 (2013-05), Seiten 424-429, XP055150075, ISSN: 0024-8525, DOI: 10.1007/s35146-013-0109-3
- Sven Krause: "Massenspektrometrisches Verfahren zur Charakterisierung der Ölverdampfung im Brennraum von Ottomotoren", , 14. April 2009 (2009-04-14), XP055149751, Gefunden im Internet: URL:http://d-nb.info/996706453/34 [gefunden am 2014-10-29]

## Beschreibung

Die Erfindung betrifft ein Mehrfach-Ölemissionsmessgerät für Kohlenwasserstoff-Emissionen, wie sie insbesondere von Motoren ausgehen. Das Mehrfach-Ölemissionsmessgerät umfasst eine Abgassonde zur Entnahme einer Probemenge, einen Messkanal mit einer Transfer-Kapillare, eine Ionenquelle sowie eine Messeinrichtung, die als Breitbandmesseinrichtung eine Globalmessung über ein Massenspektrum hinweg durchführt.

Die Verringerung von schädlichen Emissionen aus Motoren (oder anderen Wärmekraftmaschinen) spielt zur Erfüllung immer größerer Umweltschutzanstrengungen eine bedeutende Rolle. Hierbei geht es neben Emissionen aus dem Verbrennungsprozess an sich auch um Emissionen, die aus Nebenvorgängen in oder am Motor herrühren. Hierbei kann es sich sowohl um Emissionen handeln, die als Edukte des Verbrennungsprozesses anzusehen sind, oder aber auch um solche, die durch Eintrag von Ölmengen in das Abgas des Motors resultieren, sei es durch Abschabungen von Wandungen, als Dampf- oder als Tropfeneintrag. Um zu einer Verringerung der Emissionen gelangen zu können, ist es erforderlich, die Natur und die Art der Emissionen zu erfassen und zu bewerten. Dazu müssen die Ölemissionen insbesondere von unverbrannten Kohlenwasserstoffen gemessen werden, und zwar mit hoher Geschwindigkeit über einen großen Massenbereich hinweg, um so auch innermotorische Vorgänge mit ausreichender Dynamik abbilden zu können.

Insbesondere geht es um die Bestimmung von Ölemissionen, die durch verschiedene Mechanismen hervorgerufen werden. Zum einen handelt es sich hierbei um ein Abdampfen, das insbesondere für leichtflüchtigere Moleküle mit größerer Wahrscheinlichkeit auftritt als bei schwerflüchtigen, wobei dies wiederum von der thermischen Energie abhängt. Ein weiterer bedeutender Mechanismus ist eine parasitäre Strömung in dem Brennraum (reverse blow-by), die eine Ausgleichsgasströmung darstellt und im Hubkolbenmotor insbesondere im Bereich der Kolbenringe und Kolbennuten anzutreffen ist. Sie führt in den Brennraum und führt hierbei Öl in Form von Tropfen mit sich. Schließlich besteht ein weiterer bedeutender Mechanismus im Abschaben und/oder Abschleudern von Öl durch mechanische Kräfte, so dass das Öl in Form von Tropfen aus dem Öl herausgerissen wird und in den Brennraum bzw. den Abgasstrom gelangt.

Einschlägig bekannt aus dem Stand der Technik sind verschiedene Messprinzipien. Ein erstes Messprinzip basiert auf Chemi-Lumineszenz bzw. UV-Fluoreszenz zur Analyse von Ölverbrennungsrückständen und/oder Tracersubstanzen. Bei diesem Messprinzip kann der Ölverbrauch nur dann sicher gemessen werden, wenn die Ölbestandteile vollständig zu Verbrennungsrückständen umgewandelt werden, beispielsweise in Form von Schwefeldioxid SO₂. Grundsätzlich ist es so, dass im Verbrennungsabgas je nach Gemischbildungsparametern hierfür nicht zuverlässig genug ausreichend Sauerstoff bzw. thermische Energie vorhanden ist. Somit wird zur Gewährleistung einer vollständigen Verbrennung deshalb zusätzlich ein Oxidationsofen vorgesehen. Für die Verbrennung ist ein Betriebsdruck erforderlich, der nicht zu stark vom Umgebungsdruck abweichen darf. Um einen reibungslosen Probegastransfer sicherzustellen, darf dieser Betriebsdruck nur einen geringen Druckunterschied zu dem Gasentnahmeort aufweisen. Dies kann zu Einschränkungen in Bezug auf die Dynamik führen. Weiter stellt der Ofen selbst in der Messkette einen erheblichen Tiefpass dar, der die Anwendbarkeit für dynamische Messaufgaben einschränkt. Ein weiterer wesentlicher Nachteil dieses Messprinzips besteht darin, dass insbesondere Detektoren für UV-Fluoreszenz eine ausgeprägte Querempfindlichkeit gegenüber anderen Verbrennungsrückständen bzw. Abgasbestandteilen aufweisen, so dass es zu einer Verfälschung der Messergebnisse aufgrund von nicht dem eigentlichen Schmieröl zuzuordnenden Signalen führt.

Ein weiteres Messprinzip ist bekannt, bei dem das Messgas mit Massenspektrometrie auf Ölverbrennungsrückstände und/oder Tracersubstanzen hin analysiert wird. Dieses hat gegenüber dem Vorgenannten den Vorteil einer geringeren Querempfindlichkeit gegenüber weiteren Verbrennungsrückständen bzw. anderen Abgasbestandteilen. Allerdings muss es ebenso mit einem Ofen zur Nachverbrennung betrieben werden. Damit ergeben sich dieselben Nachteile in Bezug auf die Dynamik wie bei dem vorbeschriebenen Verfahren.

Ein weiteres Messprinzip basiert auf Radioaktivität. Hierbei wird ein zuvor in die Kohlenwasserstoffketten des Schmieröls eingebundenes radioaktives Element gesammelt mittels eines Filters oder einer Kondensatfalle, und es wird schließlich mittels eines Radioaktivitätsdetektors gemessen. Der Umgang mit radioaktiven Quellen verlangt besondere Sorgfalt und ist deshalb aufwendig. Zudem ist das Messergebnis beeinflusst durch die Filtercharakteristik bzw. die Fähigkeit der Kondensatfalle, ein entsprechendes Kondensat zu bilden. Ferner hat sich gezeigt, dass Öltropfen bei mangelnder thermischer Energie, wie sie insbesondere im Schubbetrieb des Motors vorkommen, nicht an den Sammelort gelangen, sondern sich bereits vorweg an Leitungswandungen ablagern. Damit tritt eine Verfälschung des Messergebnisses zu niedrigen Werten hin auf.

Ein weiteres Messprinzip ist bekannt aus der DE 10 2004 001 514, bei welchem unverbrannte Bestandteile des Schmieröls einem als elektrischen Multipol ausgeführten Hochfass-Massenfilter zugeführt und anschließend einer Massenspektrometrie unterzogen werden. Die Messeinrichtung an sich weist zwar eine hohe Dynamik auf und erfüllt insoweit die Anforderungen. Allerdings ist ihre Leistung in Bezug auf die Detektion von Ölemissionen in Tröpfchenform unbefriedigend.

Weiterhin offenbart DE 10 2010 012606 eine Vorrichtung gemäß der Präambel des Hauptanspruchs.

Ausgehend von dem zuletzt genannten Stand der Technik liegt der Erfindung die Aufgabe zugrunde, eine verbesserte Messeinrichtung zu erschaffen, welche bei weiterhin hoher Dynamik eine Betriebspunkt-robuste Erfassung auch von Ölemissionen ermöglicht, und zwar sowohl von dampfförmigen wie auch tröpfchenförmigen.

Die erfindungsgemäße Lösung liegt in den Merkmalen des unabhängigen Anspruchs. Vorteilhafte Weiterbildungen sind Gegenstand der abhängigen Ansprüche.

Bei einem Mehrfach-Ölemissionsmessgerät für Kohlenwasserstoffemissionen in einem Abgasgemisch umfassend eine Abgassonde mit einer Transferkapillare und einen Messkanal mit einer Ionenquelle, einer Filtereinrichtung mit einer Messeinrichtung, wobei die Filtereinrichtung vorzugsweise eine Einstelleinrichtung zur Bestimmung eines Durchlassbereichs einer zu messenden Schmierölfraktion aufweist, und wobei die Messeinrichtung eine Breitbandmesseinrichtung ist, die vorzugsweise eine Globalmessung der Konzentration der Moleküle in einem Schritt über den Durchlassbereich durchführt, ist erfindungsgemäß vorgesehen, dass die Transferkapillare an ihrer Spitze eine Tröpfchenfangeinrichtung aufweist, die einen kurzen Drosselabschnitt und einen in Strömungsrichtung sich anschließenden mindestens zehnmal längeren Transferabschnitt aufweist, und die Messeinrichtung mit einer Analyseeinrichtung verbunden ist, wobei die Analyseeinrichtung einen Klassifikator für dampfförmige Ölbestandteile und tröpfchenförmige Ölbestandteile umfasst.

Die Erfindung beruht auf dem Gedanken, mittels des Klassifikators eine quantifizierende Unterscheidung zwischen dampfförmigen und tröpfchenförmigen Ölemissionen zu treffen. Mittels dieses Klassifikators ist es möglich, eine Unterscheidung hinsichtlich der Dampf- bzw. Tröpfchenölemissionen durchzuführen. Daraus können Aussagen über die Entstehung der Ölemissionen getroffen werden, wodurch unnötiger Ölverbrauch erkannt und entsprechende Maßnahmen zu dessen Verringerung eingeleitet werden können. Die Klassifikation hinsichtlich dampf- bzw. tröpfchenförmiger Emissionen für sich wäre jedoch wertlos, wenn tröpfchenförmige Emissionen nicht hinreichend sicher erfasst werden. Daher sieht die Erfindung eine Kombination mit einer speziell ausgebildeten Abgassonde vor, welche aufgrund ihrer Gestaltung besonders geeignet ist zur Erfassung (auch) von tröpfchenförmigen Ölemissionen. Da es dank der guten Erfassung auch von tröpfchenförmigen Ölemissionen für die nachfolgende Bestimmung irrelevant ist, ob die Ölemissionen vom Dampf oder als Aerosol in Gestalt von Tröpfchen erfolgen, ist die erfindungsgemäße Bestimmungseinrichtung auch robust gegenüber Betriebspunktschwankungen mit der damit einhergehenden Verschiebung zwischen dampf- und tröpfchenförmigen Ölemissionen, je nach thermischer Energie. Durch die Kombination dieser Maßnahmen stellt die Erfindung somit eine dynamische, genaue und dank sicherer Erfassung von tröpfchenförmigen Emissionen auch Betriebspunkt-robuste Bestimmung der Ölemissionen bereit.

Zweckmäßigerweise ist ein Kalibrierer für den Klassifikator vorgesehen, wobei der Kalibrierer einen ersten Speicher für Referenzdaten von dampfförmigen Ölbestandteilen und einen zweiten Speicher für Referenzdaten von tropfenförmigen Ölbestandteilen aufweist. Auf diese Weise kann der Klassifikator einfach und genau an die Spektren der zu analysierenden Ölemissionsbestandteile angepasst werden. Indem entsprechende Daten in die ersten und zweiten Speicher eingespeichert werden können, kann auf diese Weise auch leicht eine Anpassung an andere Motoren bzw. andere Ölsorten erfolgen.

Vorzugsweise ist hierzu dem Kalibrierer ein Matched-Filter zugeordnet.

Zweckmäßigerweise ist das Matched-Filter zur Erkennung von dampfförmigen Ölbestandteilen bzw. tröpfchenförmigen Ölbestandteilen ausgebildet. Somit kann die Art der Schmieröl-Emission besser bestimmt werden. Hierbei kann sich das Matched-Filter Erkenntnisse zur Wichtung verschiedener Felder/Unterfelder zunutze machen. Korreliert beispielsweise ein hoher Anteil an schwerflüchtigen Schmierölbestandteilen mit einem eher mäßigen Anteil an leichtflüchtigen Schmierölbestandteilen, so spricht dies für einen Ölverlust durch Abschaben oder Abschleudern. Umgekehrt spricht ein überwiegendes Auftreten von leichtflüchtigen Schmierölbestandteilen gegenüber schwerflüchtigen Schmierölbestandteilen für eine Emission aufgrund von Abdampfen. So kann durch eine Korrelation der Anteile von leichtflüchtigen Schmierölbestandteilen mit leicht- und schwerflüchtigen Schmierölbestandteilen eine Aussage über die Art des Entstehungsmechanismus getroffen werden. Das Matched-Filter ermöglicht dies in zuverlässiger und automatisierter Weise.

Mit Vorteil ist an der Abgassonde ein zweiter Messkanal angeordnet, der an eine Bestimmungseinrichtung für verbrannte Kohlenwasserstoffe angeschlossen ist. Hiermit wird ein zweiter Messkanal geschaffen, der neben den durch die Analyseeinrichtung bestimmten unverbrannten Kohlenwasserstoffe auch die verbrannten Kohlenwasserstoffe berücksichtigt. Damit kann ein umfassendes Bild über die Ölemissionen erreicht werden. Mit Vorteil ist zu diesem Zweck ein Totalisator vorgesehen, der einen Wert für eine Gesamtemission ermittelt aus den Werten der Messeinrichtung mit der Analyseeinrichtung einerseits, wie aus den Werten der Bestimmungseinrichtung andererseits. Auf diese Weise wird eine besonders robuste Messung ermöglicht, da beispielsweise betriebspunktabhängige Verschiebungen von Ölemissionen von unverbrannten Kohlenwasserstoffen hin zu verbrannten Kohlenwasserstoffen (oder umgekehrt) durch den Totalisator erkannt werden. Eine Verfälschung des Messergebnisses aufgrund von Verbrennungsprozessen der Kohlenwasserstoffe wird damit vermieden.

Mit besonderem Vorteil ist eine Unterdruckpumpe für den Transferkanal vorgesehen. Sie ist vorzugsweise so eingestellt, dass sich eine Strömungsgeschwindigkeit von mindestens 100 m/s, vorzugsweise zwischen 130-200 m/s, an der Spitze der Tröpfchenfangeinrichtung ergibt. Dank dieser hohen Geschwindigkeit ist eine zuverlässige Erfassung von tröpfchenförmigen Emissionen gewährleistet. Dies gilt betriebspunktunabhängig, und insbesondere auch bei energiearmen Schub-Betriebspunkten, welche bei Messprinzipien gemäß dem Stand der Technik teilweise zu erheblichen Verfälschungen führten.

Die Erfindung wird nachfolgend unter Bezugnahme auf die beigefügte Zeichnung näher erläutert, in der ein vorteilhaftes Ausführungsbeispiel dargestellt ist. Es zeigen:
- Fig. 1: eine Übersichtsdarstellung einer Vorrichtung gemäß einem Ausführungsbeispiel der Erfindung;
- Fig. 2: eine Detaildarstellung einer Abgassonde mit einem Messkanal gemäß dem Ausführungsbeispiel;
- Fig. 3 a, b, c: ein Massenspektrogramm zur Veranschaulichung einer Wirkweise eines Klassifikators; und
- Fig. 4 a, b: ein Vergleichsdiagramm zu einer Abgassonde gemäß dem Stand der Technik.

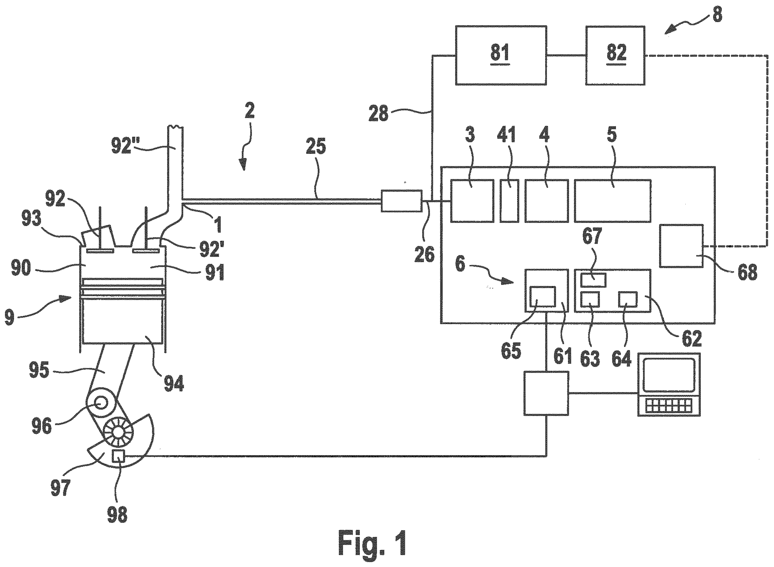

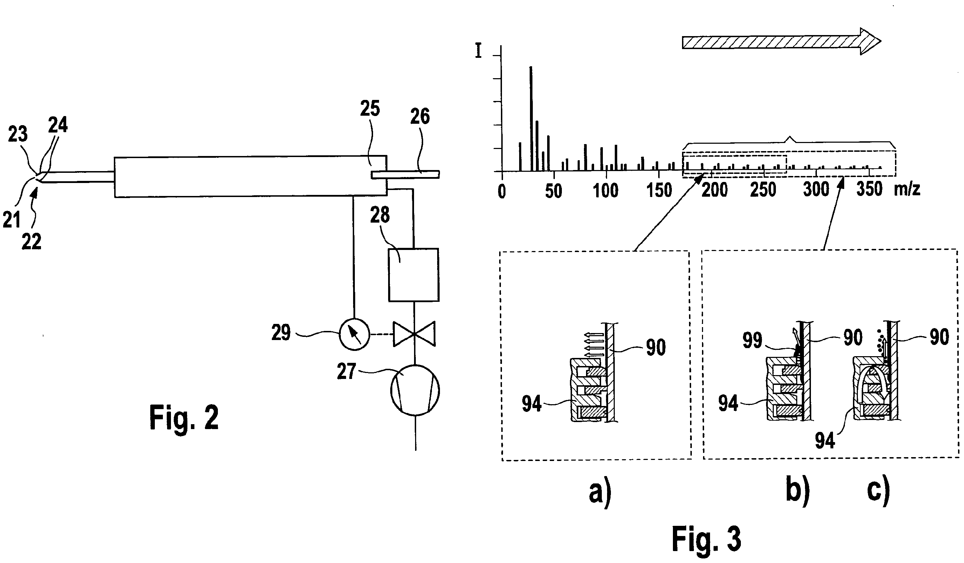

In den Figuren 1 und 2 ist ein Ausführungsbeispiel für eine Bestimmungsvorrichtung gemäß der Erfindung dargestellt. Die Bestimmungsvorrichtung dient zur Bestimmung von Ölemissionen, allgemeiner gesagt zur Bestimmung von Emissionen unverbrannter Kohlenwasserstoffe (HC), die von einem Verbrennungsmotor stammen. Beim dargestellten Ausführungsbeispiel handelt es sich um einen Verbrennungsmotor 9 nach dem Hubkolbenprinzip, jedoch ist das Ausführungsbeispiel der Erfindung nicht darauf beschränkt. Der Motor 9 umfasst mindestens einen Zylinder 90, in dem ein Kolben 94 auf und ab beweglich geführt ist. Oberhalb des Kolbens ist ein Brennraum 91 ausgebildet, dem über ein Ventil 92 Frischgas zugeführt und aus dem über ein Ventil 92' Abgas in ein Abgasrohr 92" abgeführt wird. Der Kolben 94 ist über eine Pleuelstange 95 mit einem Kurbelzapfen 96 einer Kurbelwelle 97 verbunden. An der Kurbelwelle 97 ist ein Winkelgeber 98 angeordnet, der ein Messsignal für Kurbelwellenposition und -drehzahl ausgibt.

Der Verbrennungsmotor 9 ist an sich konventionell ausgeführt, so dass auf eine detaillierte Beschreibung verzichtet werden kann. Als Besonderheit weist er am Abgasrohr 92" eine Abgassonde 1 auf, an die eine Transferkapillare 2 angeschlossen ist und die verbunden ist mit einer Filtereinrichtung 4 sowie einer Messeinrichtung 5. Bei einem alternativen, nicht dargestellten Ausführungsbeispiel führt die Abgassonde 1 durch die Wandung des Zylinderkopfs. Sie weist zum Zylinderkopf einen verengten Durchmesser auf, der sich vom Zylinderkopf weg mehrstufig verbreitert. An einem Bereich mit breitem Durchmesser ist ein Einlass der Transferkapillare 2 angeschlossen.

Die Transferkapillare 2 weist an ihrer vorderen Spitze 21, mit der sie an das Abgasrohr 92" angeschlossen ist, eine Tröpfchenfangeinrichtung 22 auf. Diese besteht aus einem unmittelbar in der Mündung angeordneten Drosselabschnitt 23, der schräg unter einem Winkel von etwa 45° entgegen der Strömungsrichtung ragende Leitbleche 24 aufweist. Die Leitbleche 24 sind dabei passend zur Innenform der Transferkapillare 2 geformt, sie weisen also bei einer kreisrunden Transferkapillare 2 insgesamt etwa die Form eines Kegelstumpfmantels auf. Typische Werte für den Durchmesser der Transferkapillare 2 betragen 0,5-2 mm, während in dem Drosselabschnitt 23 der Tröpfchenfangeinrichtung 22 der Durchmesser verengt ist auf 0,2-0,5 mm.

Der Hauptbereich der Transferkapillare 2 erstreckt sich als ein rohrartiger Transferabschnitt, der in seinem hinteren Bereich eine Druckstufe 25 mit einem erweiterten Rohrdurchmesser aufweist. Dieser beträgt etwa 2-6 mm. An das hintere Ende der Druckstufe 25 ist eine Restriktionskapillare 26 angeschlossen, die zu einem Eingangsanschluss der Filter- und Messeinrichtung 4, 5 führt. Die Restriktionskapillare 26 weist einen erheblich kleineren Durchmesser von 50-500 µm auf. Durch diese Querschnittsverminderung erfolgt eine Teilung des Massenstroms sowie eine druckmäßige Entkopplung. Der übrige Massenstrom wird abgeführt zu einer Vakuumpumpe 27, die über einen vorgeschalteten Vakuumtank 28 ebenfalls an das hintere Ende der Druckstufe 25 angeschlossen ist (s. Fig. 2). Mit einer Drucksteuerung 29 wird ein gewünschter Unterdruck mit Druckstufe 25 und in der Transferkapillare 2 aufrechterhalten.

Die von der Restriktionskapillare 26 an den Messkanal weitergeführte Probenmenge wird, bevor sie die Filtereinrichtung 4 erreicht, zuerst einer Ionenquelleneinrichtung 3 zugeführt. Diese ist dazu ausgebildet, die einströmende Gasmenge zu ionisieren. Sie ist beispielsweise als ein Ionisierer nach dem SMB-Prinzip (Supersonic Molecular Beam) ausgeführt. Unmittelbar in Flussrichtung anschließend an die Ionenquelle 3 ist die Filtereinrichtung 4 angeordnet. Ihr ist eine Einstelleinrichtung 41 zugeordnet, an der ein Massenbereich eingestellt werden kann, der von der Filtereinrichtung 4 durchzulassen ist. Die Filtereinrichtung 4 fungiert damit als ein Massenfilter, das nur Ionen in einem gewünschten Durchlassbereich des Massenspektrums passieren lässt und die übrigen ausfiltert. Die Filtereinrichtung 4 kann beispielsweise als Quadropol-Filter ausgebildet sein. Der Aufbau von Quadropol-Filtern im Allgemeinen ist im Stand der Technik bekannt und braucht hier nicht mehr erläutert zu werden.

Weiter in Flussrichtung hinter der Filtereinrichtung 4 ist die Messeinrichtung 5 angeordnet. Die Messeinrichtung 5 umfasst einen Detektor und einen Massenseparator. Diese Messeinrichtung ermöglicht eine breitbandige quasisimultane Messung der Intensitätsverteilung der Ionen über den spezifizierten Durchlassbereich hinweg. Das so gewonnene Messsignal ist ein Intensitätsfolgesignal und wird an eine Analyseeinrichtung 6 übermittelt. Die Messeinrichtung 5 ist dazu befähigt, das komplette Spektrum über den gewünschten Durchlassbereich durch schnelles Scannen mit hoher Dynamik und Auflösung zu erfassen.

Die Analyseeinrichtung 6 umfasst einen Klassifikator 61, der mit einem Kalibrierer 62 zusammenwirkt. Der Kalibrierer 62 umfasst einen ersten Speicher 63 sowie einen zweiten Speicher 64. Im ersten Speicher 63 sind Referenzdaten zur spektralen Verteilung von dampfförmigen Ölbestandteilen und im zweiten Speicher 64 Referenzdaten zur spektralen Massenverteilung von tropfenförmigen Ölbestandteilen enthalten. Damit kann durch den Kalibrierer 62 der Klassifikator 61 so eingestellt werden, dass er in den Messergebnissen der Messeinrichtung 5 die dampfförmigen Ölbestandteile einerseits und die tropfenförmigen Ölbestandteile andererseits unterscheidet und auswertet.

Vorzugsweise ist zur Unterscheidung von dampfförmigen und tropfenförmigen Ölbestandteilen die Bildung von Unterfeldern vorgesehen. Ein derartiges Unterfeld ist in Figur 3 in der oberen Darstellung gezeigt. Es sind zwei Unterfelder mit strichlinierten Linien dargestellt, ein größeres (im Bereich von 170-380 m/z) und ein kleineres (im Bereich von 170-270 m/z). Es sei angemerkt, dass in diesem Fall die Unterfelder überlappen, sie können aber auch getrennt voneinander sein. Das kleine Unterfeld steht hierbei für die leichtflüchtige Fraktion, während das große Unterfeld für die Summe aus leicht- und schwerflüchtigen Fraktionen steht.

Zur Auswertung, insbesondere unter Gewichtung von verschiedenen einstellbaren Feldern bzw. Unterfeldern, ist zweckmäßigerweise ein Matched-Filter 65 vorgesehen. Es bildet einen Teil des Klassifikators 61 und wirkt ebenfalls mit dem Kalibrierer 62 zusammen, um so eine Zuordnung der ermittelten Massenspektren unter Berücksichtigung der Intensitätsfolgen zu vorbestimmten Entstehungsmechanismen durchzuführen. Hiermit kann beispielsweise ermittelt werden, ob die gemessenen Schmieröl-Emissionen beruhen auf einfachem Abdampfen oder auf mechanischen Vorgängen, wie Abschaben oder Abschleudern des Öls von der Innenwand des Zylinders 90. Dies sei für eine Schmierölsorte beispielhaft anhand der Figur 3 a-c erläutert (in anderen Fällen können sich abweichende Massengrenzen ergeben). Zu dieser Bestimmung macht man sich die Erkenntnis zunutze, dass leicht- und schwerflüchtige Schmieröl-Fraktionen in der Regel auf verschiedene Weise in die Schmieröl-Emissionen gelangen. Die leichtflüchtigen Schmieröl-Fraktionen gelangen in der Regel in das Schmieröl durch Abdampfen, insbesondere von der Innenwand des Zylinders (s. Fig. 3 a entsprechend das mit der grau strichlierten Linie umrandete kleine Unterfeld in dem darüber angeordneten Diagramm). Entstehen die Schmierstoff-Emissionen hingegen durch Abschaben an der Innenwand des Zylinders 90, beispielsweise durch Ölkohle 99 auf dem Kolben 91 (s. Fig. 3 b), so ist die Schmieröl-Emission im Spektrum breitbandiger und reicht bis über Molekülionen mit einer spezifischen Ladung von über 350 m/z hinaus (s. das durch die schwarz strichlierte Linie umrandete Unterfeld). Für ein Abschleudern des Öls, insbesondere an Kolbenringen des Kolbens 94 (s. Fig. 3 c) wird im Prinzip Ähnliches verdeutlicht: Auch hierbei gelangen sowohl leicht- wie auch schwerflüchtige Schmieröl-Fraktionen ins Abgas und werden dann als Schmieröl-Emission erfasst. Durch eine Korrelation der Anteile von leichtflüchtigen Schmieröl-Fraktionen verglichen mit leicht- und schwerflüchtigen Schmieröl-Fraktionen kann sogar eine Aussage über die Art des Entstehungsmechanismus getroffen werden. Dieser erfolgt automatisiert mittels des Matched-Filters.

Die Bestimmungsvorrichtung weist noch einen weiteren Messzweig 8 auf zur Bestimmung von Verbrennungsrückständen. Er umfasst ein an sich bekanntes Massenspektrometer 81 mit angeschlossener Messeinrichtung 82. Diese übermittelt die gewonnenen Messdaten an ein Interface-Modul 68 der Analyseeinrichtung 6. Damit kann die Analyseeinrichtung 6 bei der Auswertung auch Daten zu verbrannten Ölbestandteilen berücksichtigen. Dies ermöglicht zum einen ein umfassenderes Messergebnis und damit einen Überblick über die gesamten Emissionen des Öls, seien sie verbrannt oder unverbrannt. Zum anderen kann damit die Bestimmung des Ölemissionsmechanismus noch genauer erfolgen, da es insbesondere bei Öldämpfen häufig zu einer mindestens teilweisen Verbrennung der Ölemission kommt, die somit dank des zusätzlichen Messzweiges 8 erfasst und mittels des Interface 68 bei der weiteren Auswertung berücksichtigt wird. Der zweite Messzweig 8 ist über einen zweiten Messkanal 28 an die Transferkapillare 2 angeschlossen.

Weiter ist ein Totalisator 67 vorgesehen. Dieser dient dazu, unter Zugrundelegung der Messwerte von der Messeinrichtung 6 für die unverbrannten Kohlenwasserstoff-Emissionen und der von dem Interface 68 stammenden Messwerte für die verbrannten Ölemissionen einen Gesamtwert zu bilden.

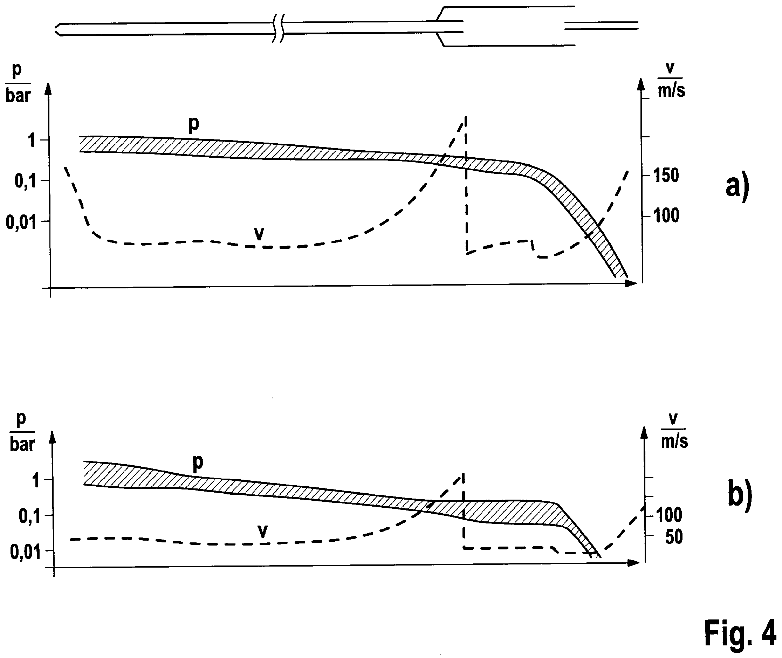

Zur Bildung und Aufrechterhaltung des erforderlichen Unterdrucks in der Transferkapillare 2 ist die Unterdruckpumpe 27 und die Drucksteuerung 29 vorzugsweise dazu ausgebildet, einen Unterdruck von etwa 0,3 bar absolut zu erzeugen. Aufgrund der geometrischen Verhältnisse in der Transferkapillare 2 mit der Druckstufe 25 und der Leitung 22 ergibt sich ein Druckverlauf, wie in Figur 4 a dargestellt ist. Weiter ist mit gestrichelter Linie in Figur 4 a dargestellt die sich bei dem Druckverlauf einstellende Strömungsgeschwindigkeit in der Transferkapillare 2 an den jeweiligen Stellen. Man erkennt, dass mit dem entsprechend eingestellten Druck an dem Drosselabschnitt 23 der Tropfenfangeinrichtung 22 eine sehr hohe Strömungsgeschwindigkeit von etwa 150 m/s erreicht wird. Dies ist eine erhebliche Steigerung gegenüber bekannten Abgassonden aus dem Stand der Technik, von der ein Vergleichsbild in Figur 4 b dargestellt ist. Hierbei ergibt sich bei gleicher Unterdruckbeaufschlagung am Ende der Transferkapillare 2 lediglich eine Geschwindigkeit (s. gestrichelte Linie) an der Spitze der Transferkapillare von weniger als 50 m/s. Mit der erfindungsgemäßen Tropfenfangeinrichtung 22 kann dank des Drosselabschnitts 23 so auf verblüffend einfache und effiziente Weise eine Verdreifachung der Eintrittsgeschwindigkeit erreicht werden, was eine Verneunfachung der Energie bedeutet, mit welcher die Tröpfchen mitgerissen werden. Damit verbessert sich die Fähigkeit der Sonde zum "Einfangen" der Tröpfchen erheblich, so dass die Qualität der Messergebnisse entsprechend deutlich gesteigert ist.

## Patentansprüche

1. Mehrfach-Ölemissionsmessgerät für Kohlenwasserstoffemissionen in einem Abgasgemisch umfassend eine Abgassonde (1) mit einer Transferkapillare (2), die ausgebildet ist zur Anordnung an einem Abgasrohr (92") eines Verbrennungsmotors (9), und einen Messkanal mit einer Ionenquelle (3) und einer Filtereinrichtung (4) mit einer Messeinrichtung (5), wobei die Filtereinrichtung (4) vorzugsweise eine Einstelleinrichtung (41) zur Bestimmung eines Durchlassbereichs gemäß einer zu messenden Schmieröl-Fraktion aufweist, wobei die Messeinrichtung (5) eine Breitbandmesseinrichtung ist, die vorzugsweise eine Globalmessung der Konzentration der Moleküle in einem Schritt über den Durchlassbereich durchführt,
**dadurch gekennzeichnet, dass**
die Transferkapillare (2) an ihrer Spitze (21), die ausgebildet ist zum Anschluss an das Abgasrohr (92"), mit einer Tröpfchenfangeinrichtung (22) versehen ist, die einen in der Mündung an der vorderen Spitze (21) angeordneten kurzen Drosselabschnitt (23) und einen in Strömungsrichtung sich anschließenden mindestens zehnmal längeren Transferabschnitt aufweist, und die Messeinrichtung (5) mit einer Analyseeinrichtung (6) verbunden ist, die einen Klassifikator (61) umfasst, der ausgebildet ist für eine quantifizierende Unterscheidung zwischen dampfförmigen Ölbestandteilen und tropfenförmigen Ölbestandteilen.

2. Bestimmungsvorrichtung nach Anspruch 1, **dadurch gekennzeichnet, dass** ein Kalibrierer (62) für den Klassifikator (61) vorgesehen ist, wobei der Kalibrierer (62) einen ersten Speicher (63) für Referenzdaten von dampfförmigen Ölbestandteilen und einen zweiten Speicher (64) für Referenzdaten von tropfenförmigen Ölbestandteilen aufweist.

3. Bestimmungsvorrichtung nach Anspruch 1 oder 2, **dadurch gekennzeichnet, dass** dem Klassifikator (61) ferner ein Matched-Filter (65) zugeordnet ist, das zur Erkennung von dampfförmigen Ölbestandteilen und tropfenförmigen Ölbestandteilen ausgebildet ist.

4. Bestimmungsvorrichtung nach einem der vorangehenden Ansprüche, **dadurch gekennzeichnet, dass** an Z der Abgassonde (1) ein zweiter Messkanal (28) angeordnet ist, der an eine Bestimmungseinrichtung (8) für verbrannte Kohlenwasserstoffe angeschlossen ist.

5. Bestimmungsvorrichtung nach Anspruch 4, **dadurch gekennzeichnet, dass** ein Totalisator (68) vorgesehen ist, der einen Wert für einen Gesamtverbrauch ermittelt aus den Werten der Analyseeinrichtung (6) und der Bestimmungseinrichtung (8).

6. Bestimmungsvorrichtung nach einem der vorangehenden Ansprüche, **dadurch gekennzeichnet, dass** eine Unterdruckpumpe für den Transferkanal (2) vorgesehen ist, die vorzugsweise so eingestellt ist, dass sie eine Strömungsgeschwindigkeit von mindestens 100 m/s, vorzugsweise 130-200 m/s, an der Spitze der Tröpfchenfangeinrichtung (22) erzeugt.

## Claims

1. Multiple oil-emissions meter for hydrocarbon emissions in an exhaust-gas mixture comprising an exhaust-gas probe (1) with a transfer capillary (2) which is designed for being arranged on an exhaust pipe (92") of an internal combustion engine (9) and comprising a measuring channel with an ion source (3) and a filtering device (4) with a measuring device (5), the filtering device (4) preferably having a setting device (41) for determining a passband range according to a lubricating oil fraction to be measured, the measuring device (5) being a broadband measuring device, which preferably carries out a global measurement of the concentration of the molecules in one step over the passband range,
**characterized in that**
the transfer capillary (2) is provided at its tip (21), which is designed for being connected to the exhaust pipe (92''), with a droplet-catching device (22), which has a short throttle segment (23), which is arranged in the mouth at the front tip (21), and a transfer segment, which adjoins the throttle segment in the direction of flow and is at least ten times longer, and the measuring device (5) is connected to an analysing device (6), which comprises a classifier (61), which is designed for quantifying differentiation between oil constituents in the form of vapours and oil constituents in the form of droplets.

2. Determining device according to Claim 1, **characterized in that** a calibrator (62) is provided for the classifier (61), the calibrator (62) having a first memory (63) for reference data of oil constituents in the form of vapours and a second memory (64) for reference data of oil constituents in the form of droplets.

3. Determining device according to Claim 1 or 2, **characterized in that** the classifier (61) is also assigned a matched filter (65), which is designed for the detection of oil constituents in the form of vapours and oil constituents in the form of droplets.

4. Determining device according to one of the preceding claims, **characterized in that** arranged on the exhaust-gas probe (1) is a second measuring channel (28), which is connected to a determining device (8) for combusted hydrocarbons.

5. Determining device according to Claim 4, **characterized in that** a totalizer (68), which determines a value for an overall consumption from the values of the analysing device (6) and the determining device (8), is provided.

6. Determining device according to one of the preceding claims, **characterized in that** a vacuum pump is provided for the transfer channel (2), preferably set in such a way that it generates a flow velocity of at least 100 m/s, preferably 130-200 m/s, at the tip of the droplet-catching device (22).

## Revendications

1. Appareil de mesure d'émissions d'huile multiples pour des émissions d'hydrocarbures dans un mélange de gaz d'échappement, comprenant une sonde de gaz d'échappement (1) avec un capillaire de transfert (2), qui est configuré pour être agencé sur un tuyau d'échappement (92") d'un moteur à combustion interne (9), et un canal de mesure avec une source d'ions (3) et un appareil de filtration (4) avec un appareil de mesure (5), l'appareil de filtration (4) comprenant de préférence un appareil de réglage (41) pour déterminer une plage de passage en fonction d'une fraction d'huile lubrifiante à mesurer, l'appareil de mesure (5) étant un appareil de mesure à large bande qui effectue de préférence une mesure globale de la concentration des molécules en une étape sur la plage de passage,
**caractérisé en ce que**
le capillaire de transfert (2) est muni à sa pointe (21), qui est configurée pour être raccordée au tuyau d'échappement (92"), d'un appareil de capture de gouttelettes (22) qui présente une courte section d'étranglement (23) agencée dans l'embouchure à la pointe avant (21) et une section de transfert au moins dix fois plus longue qui s'y raccorde dans la direction d'écoulement, et l'appareil de mesure (5) est relié à un appareil d'analyse (6), qui comprend un classificateur (61), qui est configuré pour une distinction quantifiante entre les constituants de l'huile sous forme de vapeur et les constituants de l'huile sous forme de gouttes.

2. Dispositif de détermination selon la revendication 1, **caractérisé en ce qu'**un calibreur (62) est prévu pour le classificateur (61), le calibreur (62) ayant une première mémoire (63) pour les données de référence de constituants de l'huile sous forme de vapeur et une deuxième mémoire (64) pour les données de référence de constituants de l'huile sous forme de gouttes.

3. Dispositif de détermination selon la revendication 1 ou 2, **caractérisé en ce que** le classificateur (61) est en outre associé à un filtre adapté (65), qui est configuré pour la détection de constituants de l'huile sous forme de vapeur et de constituants de l'huile sous forme de gouttes.

4. Dispositif de détermination selon l'une quelconque des revendications précédentes, **caractérisé en ce qu'**un un deuxième canal de mesure (28) est agencé sur la sonde de gaz d'échappement (1), qui est raccordé à un appareil de détermination (8) pour les hydrocarbures brûlés.

5. Dispositif de détermination selon la revendication 4, **caractérisé en ce qu'**un totalisateur (68) est prévu, qui détermine une valeur pour une consommation totale à partir des valeurs de l'appareil d'analyse (6) et de l'appareil de détermination (8).

6. Dispositif de détermination selon l'une quelconque des revendications précédentes, **caractérisé en ce qu'**une pompe à vide est prévue pour le canal de transfert (2), qui est de préférence réglée de telle sorte qu'elle produit une vitesse d'écoulement d'au moins 100 m/s, de préférence de 130 à 200 m/s, à la pointe de l'appareil de capture de gouttelettes (22).
